# EUROPEAN PATENT SPECIFICATION

(11) **EP 1 649 799 B1**
(45) Date of publication and mention of the grant of the patent: **29.08.2012**
(21) Application number: 04748032.2
(22) Date of filing: 22.07.2004
(51) Int. Cl.: A61B 1/018, A61B 1/005

(54) **ENDOSCOPE**
ENDOSKOP
ENDOSCOPE

(30) Priority: 28.07.2003 JP 2003202492
(43) Date of publication of application: 26.04.2006
(73) Proprietor: Olympus Corporation, Tokyo 151-0072 (JP)
(72) Inventor: MORIYAMA, Hiroki, Shibuya-ku, Tokyo 151-0072 (JP)
(74) Representative: von Hellfeld, Axel
(86) International application number: PCT/JP2004/010767
(87) International publication number: WO 2005/009229

(56) References cited:
- JP-A- 2 271 817
- JP-A- 2001 190 493
- JP-A- 2002 209 832
- JP-B1- 51 035 794
- US-A- 5 299 562
- US-A- 5 538 496
- US-A1- 2002 091 304

## Description

### Technical Field

The present invention relates to an endoscope comprising an inserting portion having a bending section which is bent in a plurality of directions.

### Background Art

Conventionally, an endoscope is widely used. With the endoscope, an elongated inserting portion is inserted in the body cavity, thereby observing the organ in the body cavity or performing various therapeutic treatments with a treatment instrument inserted in a treatment instrument inserting channel if necessary.

For example, Japanese Examined Patent Application Publication No. 51-35794 suggests one of the above-mentioned conventional endoscopes, comprising an inserting portion having a bending section which is bendable in a plurality of directions, e.g., up, down, left, and right directions.

In the endoscope disclosed in Japanese Examined Patent Application Publication No. 51-35794, the bending section is formed so that the length of a bent part thereof varies depending on the bending directions so as to select the bending radius in accordance with the situation, for example, it is largely bent in one direction or small bent in another direction.

Generally, in the endoscope, a bending operation member such as a knob or lever provided for a control section is operated, thus, a bending operation wire is pulled or loosened, and the bending operation of the bending section is performed. Further, the endoscope comprises an objective optical system and a treatment instrument inserting channel, at a distal-end portion thereof which is arranged on the distal-end side of the bending section.

Then, for example, upon the treatment with the reverse video image by the endoscope (having the bending section thereof bent by 180° or more) in a lumen of a large intestine or the like, the bending section is small bent. In this case, it is convenient because easy operation and observation by the endoscope is ensured if the treatment instrument inserting channel is in the vicinity of the target part such as tumor or the like and the objective optical system is far from the target part.

The Japanese Examined Patent Application Publication No. 51-35794 mentioned above, however, has failed to describe the relation of the positional relation of the objective optical system and the treatment instrument inserting channel and the direction of bending of the bending section, i.e. in concrete in which direction the bending section should bend with respect to the objective optical system and the treatment instrument inserting channel.

US 5,538,496 relates to a covering endoscope having a curving portion composed of a plurality of curving pieces and an observation optical system and an endoscope cover having an inserting path for inserting the endoscope and a contained unit provided along the inserting path. A side of the curving pieces facing the contained unit is formed of flat surfaces and a coupling portion coupling adjacent ones of the curving pieces are provided in a position near the flat surfaces from a center of the endoscope.

The present invention which has been achieved under such circumstances as mentioned above is to provide an endoscope which ensures good treatment work by selecting the bending radius upon the treatment with the reverse video image.

### Disclosure of Invention

The present invention is defined by claim 1.

### Brief Description of the Drawings

Fig. 1 is a diagram showing the structure of an endoscope according to an embodiment of the present invention;
Fig. 2 is a front view showing a distal-end portion shown in Fig. 1;
Fig. 3 is a perspective view schematically showing the structure of a bending section shown in Fig. 1;
Fig. 4 is a diagram showing the bending section shown in Fig. 3 with an arrow in a direction B;
Fig. 5 is a diagram showing the bending section shown in Fig. 3 with an arrow in a direction C;
Fig. 6 is a diagram showing the endoscope shown in Fig. 1 with an arrow in a direction A;
Fig. 7 is an explanatory diagram schematically showing a direction of an observed image displayed on a monitor;
Fig. 8 is an explanatory diagram schematically showing a state of an inserting portion when its distal-end portion reaches near a target part in the lumen;
Fig. 9 is an explanatory diagram schematically showing a state of the inserting portion when the bending section is bent with a small bending radius from the state shown in Fig. 8;
Fig. 10 is an explanatory diagram schematically showing a state of the inserting portion when the bending section is bent with the small bending radius at a winding part of the lumen; and
Fig. 11 is an explanatory diagram schematically showing a state of the inserting portion when the bending section is bent with a large bending radius at the winding part of the lumen.

### Best Mode for Carrying Out the Invention

Now, the embodiment in accordance with the present invention will be described with reference to the accompanying drawings.

Hereinbelow, an embodiment of the present invention will be described with reference to the drawings.

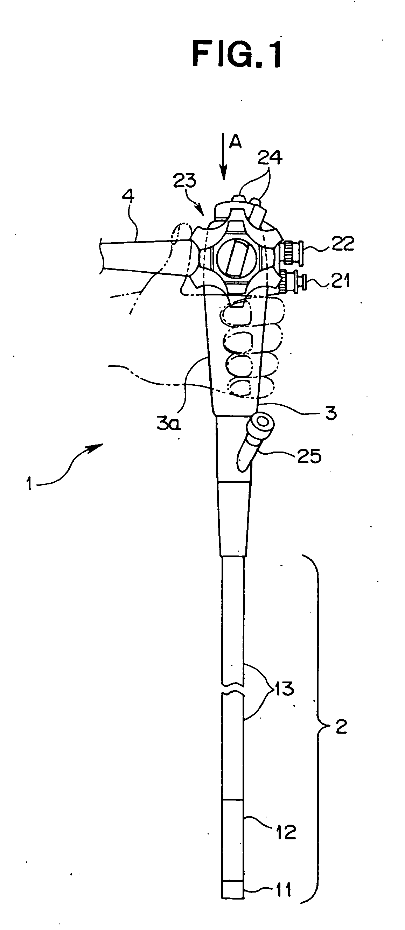

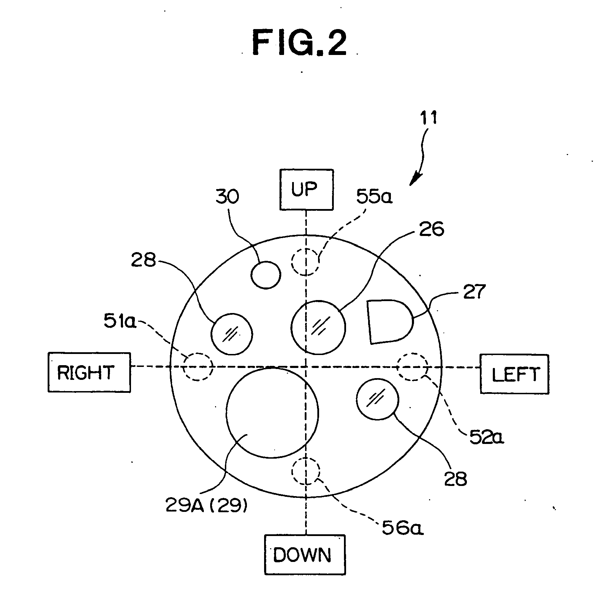

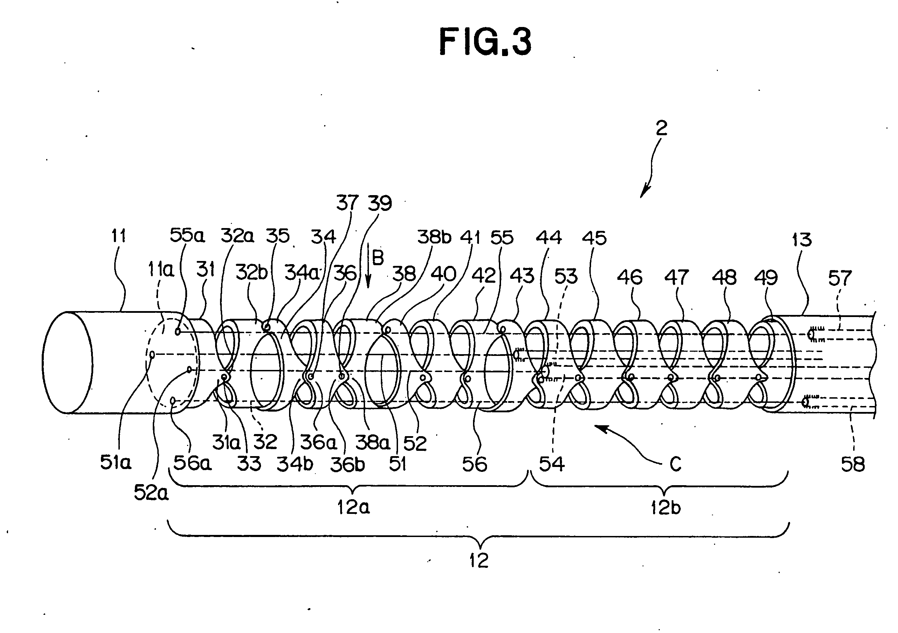

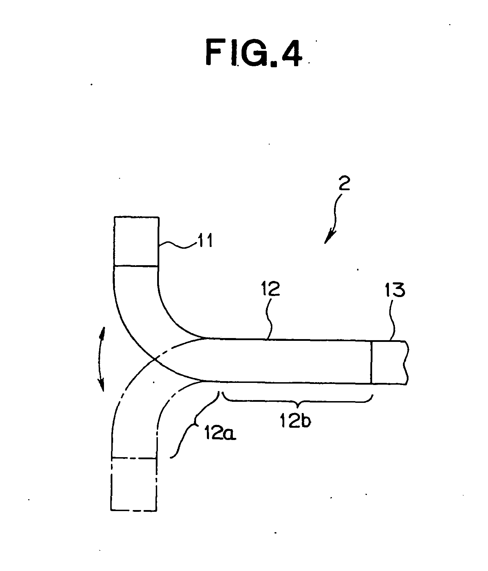

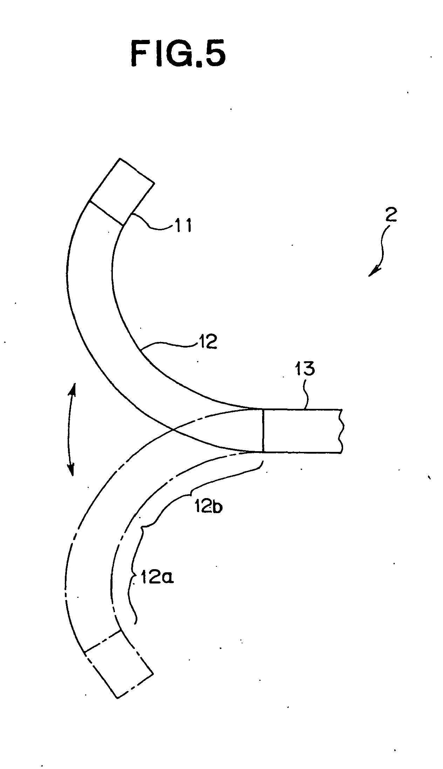

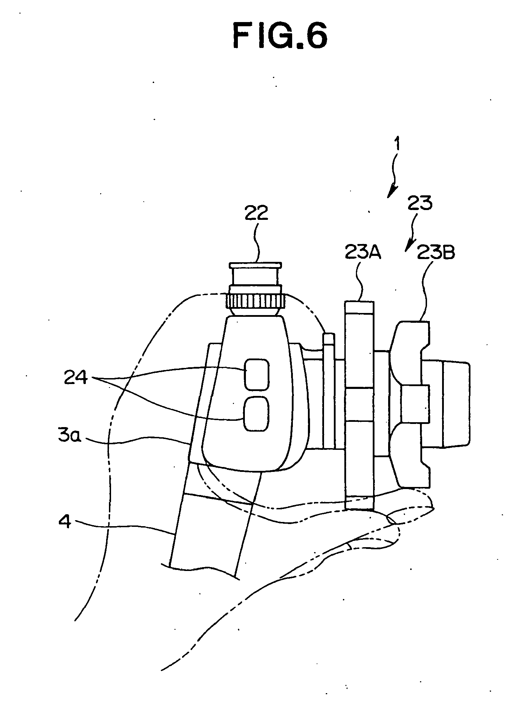

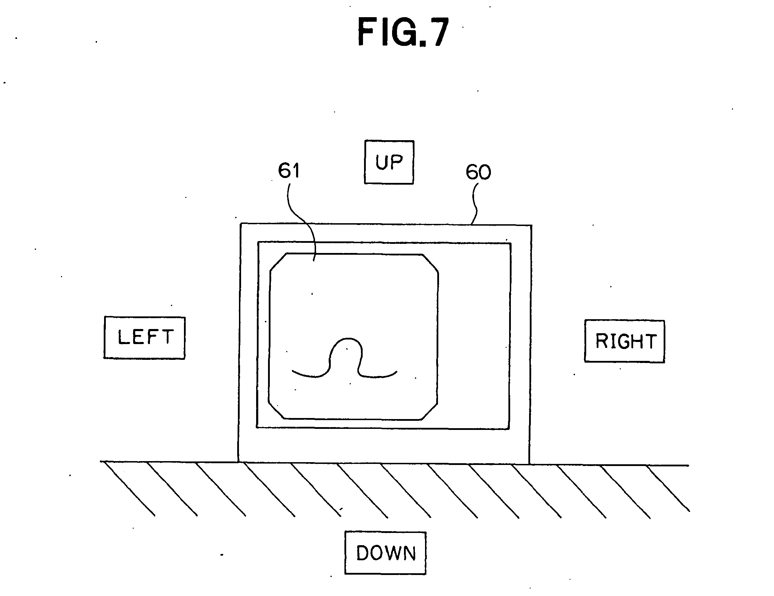

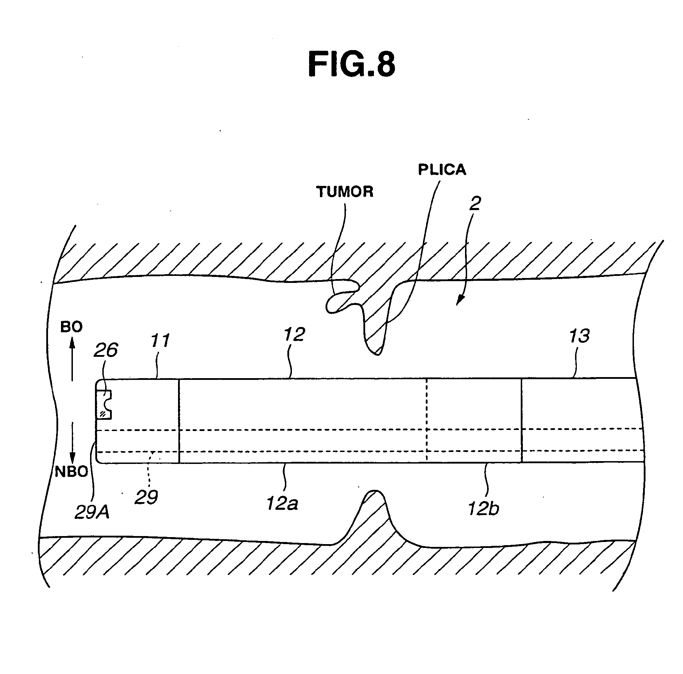

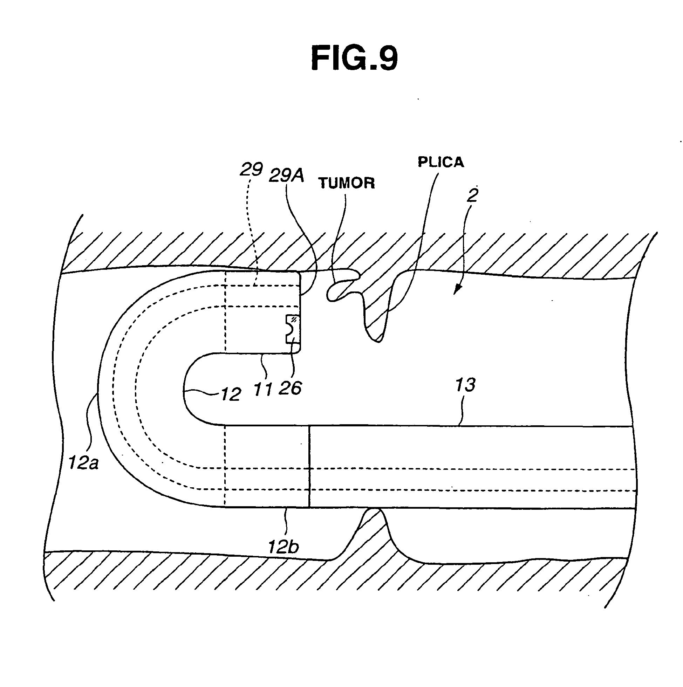

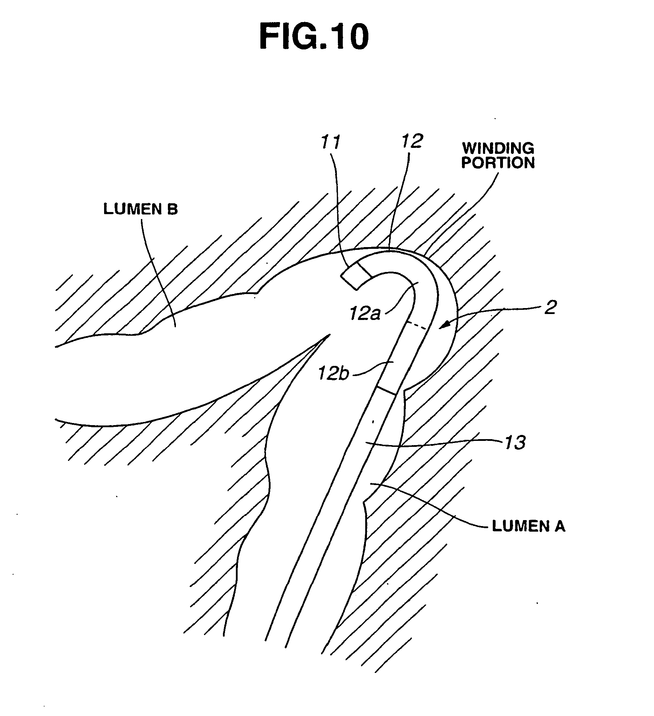

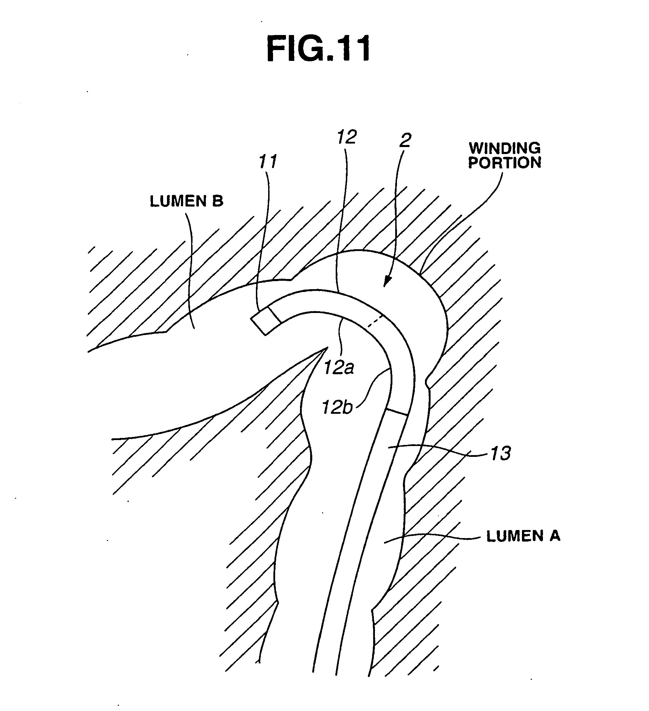

Figs. 1 to 11 relate to one embodiment of the present invention. Fig. 1 is a diagram showing the structure of an endoscope according to the embodiment of the present invention. Fig. 2 is a front view showing a distal-end portion shown in Fig. 1. Fig. 3 is a perspective view schematically showing the structure of a bending section shown in Fig. 1. Fig. 4 is a diagram showing the bending section shown in Fig. 3 with an arrow in a direction B. Fig. 5 is a diagram showing the bending section shown in Fig. 3 with an arrow in a direction C. Fig. 6 is a diagram showing the endoscope shown in Fig. 1 with an arrow in a direction A. Fig. 7 is an explanatory diagram schematically showing a direction of an observed image displayed on a monitor. Fig. 8 is an explanatory diagram schematically showing a state of an inserting portion when its distal-end portion reaches near a target part in the lumen. Fig. 9 is an explanatory diagram schematically showing a state of the inserting portion when the bending section is bent with a small bending radius from the state shown in Fig. 8. Fig. 10 is an explanatory diagram schematically showing a state of the inserting portion when the bending section is bent with the small bending radius at the winding part of the lumen. Fig. 11 is an explanatory diagram schematically showing a state of the inserting portion when the bending section is bent with a large bending radius at the winding part of the lumen.

Referring to Fig. 1, an endoscope 1 according to the embodiment comprises: an inserting portion 2 which is elongated and has flexibility; and a control section 3 which is arranged on the proximal-end side of the inserting portion 2. Further, the endoscope 1 comprises a universal cord 4 which is extended from its side portion to the control section 3 and which is flexible. The universal cord 4 comprises at its end portion a connector portion (not shown) which is detachably connected to the video processor and the light source device. Although not shown, a suction device, a frontward water-feeding device, and a liquid feed tank are connected to the endoscope 1.

The inserting portion 2 comprises: a distal-end portion 11 which is arranged to the distal end of the inserting portion 2 and which is hard; a bending section 12 which is arranged at the proximal end of the distal-end portion 11; and a flexible tube section 13 which is arranged at the proximal end of the bending section 12 and which is long and flexible.

The control section 3 comprises a grip section 3a for gripping the endoscope 1 on the side of the inserting portion 2. On the up side of the grip section 3a (on the proximal-end side of the control section 3), an air/water feed operation button 21 for operating the air/water feed operation and a suction operation button 22 for suction operation are arranged.

On the side portion of the grip section 3a to which the air/water feed operation button 21 and the suction operation button 22 are arranged, a plurality of bending operation members 23 such as a knob and a lever are arranged to perform the bending operation of the bending section 12 in a plurality of directions. According to the embodiment, as the plurality of bending operation members 23, as which will be described later, two bending operation members are arranged to bend the bending section 12 in four directions of up, down, left, and right directions. The detail will be described later.

A plurality of remote switches 24 are arranged to the top side of the grip section 3a so as to remotely control the video processor.

On the down side of the grip section 3a (proximal-end side of the inserting portion 2), a treatment instrument inserting port 25 is arranged as an opening which is communicated with a treatment instrument inserting channel 29 (refer to Figs. 8 and 9). A biopsy valve (not shown) is detachably attached to an edge portion of the treatment instrument inserting port 25.

Referring to Fig. 2, the distal-end portion 11 of the inserting portion 2 comprises: an objective optical system 26; an air/water nozzle 27 through which a fluid such as air or water spouts out to the surface of the objective optical system 26 to clean the surface of the objective optical system 26; an illuminating optical system 28; an instrument channel outlet 29A of the treatment instrument inserting channel 29; and a frontward water-feeding opening 30 through which a target part of an object is cleaned. On the back side of the objective optical system 26, an objective optical system (not shown) is arranged. Although not shown, an imaging surface of an image pick-up unit (image entry surface of an image transmitting optical system such as an image guide or a relay lens in the case of an optical endoscope) is arranged at the imaging location of the objective optical system. On the back side of the illuminating optical system 28, an output surface of a light guide (not shown) is arranged. A dotted line is a bending operation wire fixed to the distal-end portion 11, which will be described later.

According to the embodiment, at the distal-end portion 11, the treatment instrument inserting channel 29 is arranged in the outer peripheral direction of the objective optical system 26.

Next, a detailed structure of the bending section 12 will be described.

Referring to Fig. 3, the bending section 12 comprises a plurality of bending pieces (sectional rings) which are rotatably and continuously arranged. The plurality of bending pieces are covered with a bending braid which is formed by weaving thin wires (not shown) cylindrically and a bending rubber is watertightly covered on the bending braid, thereby forming the bending section 12. Fig. 3 shows the state that the bending braid and bending rubber are detached from the bending section 12.

The bending section 12 comprises two sections of a section 12a on the distal-end side and the section 12b on the proximal-end side. At the section 12a on the distal-end side, a bending piece 31 is attached to the distal-end portion 11. The bending piece 31 has a pair of pivoting portions 31a at a circumferential portion of the back end thereof (end on the proximal-end side thereof) in the horizontal direction. In the description according to the embodiment, the horizontal direction denotes a substantially lateral direction on the sheet shown in Fig. 3, for the purpose of a brief description. Further, the horizontal direction denotes one direction perpendicular to the axis of the bending section 12, and the vertical direction denotes a direction perpendicular to the horizontal direction and to the axis of the bending section 12.

At the section 12a on the distal-end side, a distal end of a bending piece 32 (hereinafter, referred to as a front end) is attached to a proximal end of the bending piece 31 (hereinafter, referred to as a back end). The bending piece 32 has a pivoting portion 32a at the circumferential portion of the front end in the horizontal direction, and further has a pivoting portions 32b at the circumferential portion of the back end in the vertical direction. The bending piece 32 can be pivoted in the up and down directions by connecting, with a pivot 33, the pivoting portion 32a to the pivoting portion 31a of the bending piece 31.

A bending piece 34 has a pivoting portion 34a at the circumferential portion of the front end in the vertical direction and a pivoting portion 34b at the circumferential portion of the back end in the horizontal direction. At the section 12a on the distal-end side, the pivoting portion 34a is connected, with a pivot 35, to the pivoting portion 32b of the bending piece 32, and thus the bending piece 34 is connected to the bending piece 32 to be pivotable in the horizontal direction.

A bending piece 36 has pivoting portions 36a and 36b at the circumferential portion of the front and back ends in the horizontal direction. The pivoting portion 36a is connected, with a pivot 37, to the pivoting portion 34b of the bending piece 34, and thus the bending piece 36 is connected to the bending piece 34 to be pivotable in the up and down directions.

Similarly, a bending piece 38 has a pair of pivoting portions 38a at the circumferential portion of the front end in the horizontal direction, and further has a pair of pivoting portions 38b at the circumferential portion of the back end in the vertical direction. The pivoting portions 38a are connected, with a pivot 39, to the pivoting portion 36b of the bending piece 36, and thus the bending piece 38 is connected to the bending piece 36 to be pivotable in the up and down directions. Similarly to the bending pieces 34, 36, and 38, a bending piece 40 is pivotably attached to the bending piece 38 in the horizontal direction. A bending piece 41 is pivotably attached to the bending piece 40 in the up and down directions. A bending piece 42 is pivotably attached to the bending piece 41 in the up and down directions. A bending piece 43 is pivotably attached to the bending piece 42 to be pivotable in the horizontal direction. As mentioned above, the bending pieces 40 to 43 are pivotably connected to the previous bending pieces alternately in the up/down directions and the horizontal direction.

The number of bending pieces alternately and pivotably connected at the section 12a on the distal-end side in the bending section 12 is not limited. Further, the bending piece 36 and the bending piece 41 may be omitted, and the bending pieces 34 and 38 may directly be connected to the bending pieces 40 and 42.

With the above-mentioned structure, the section 12a on the distal-end side of the bending section 12 is bendable in the four directions of the up and down directions and the horizontal direction.

Unlike the section 12a on the distal-end side, at the section 12b on the proximal-end side of the bending section 12, the bending pieces 44 to 49 have pivoting portions at the circumferential portion of the front and back ends in the horizontal direction, are connected to the adjacent ones with the pivoting portions, and is bendable only in the two directions, that is, up and down directions. Of course, the number of bending pieces is not limited. The bending piece 43 at the position the nearest to the proximal end of the section 12a on the distal-end side is connected to the bending piece 44 by connecting a pair of pivoting portions arranged at the circumferential portion at the back end in the horizontal direction to a pair of pivoting portions arranged at circumferential portion of a front end 38a in the horizontal direction of the bending piece 44 at the position of the most distal end of the section 12b on the proximal-end side.

A pair of bending operation wires (also referred to as strings) 51 and 52 are pulled and loosened, thus to be bent. At the section 12a on the distal-end side of the bending section 12, the pair of bending operation wires 51 and 52 pass through coil pipes (also referred to as close coils) 53 and 54, and are pierced through the bending section 12 and the flexible tube section 13 along a close portion of the pivot in the horizontal direction. The coil pipe according to the embodiment has a non-compressing structure that the wire is closely wound like a pipe.

Distal-end portions of the bending operation wires 51 and 52 are fixed at two points 51a and 52a which are apart from each other in the horizontal direction of a frame member 11a on the proximal-end side of the distal-end portion 11. Proximal-end portions of the bending operation wires 51 and 52 are connected to one bending operation mechanism (manually control mechanism) (not shown) arranged in the control section, and thus the bending operation wires 51 and 52 are alternately pulled or loosened. The one bending operation mechanism (manually control mechanism) is connected to a bending operation member 23B, which will be described later.

The coil pipes 53 and 54 are fit into the bending operation wires 51 and 52, respectively. Distal-end portions of the coil pipes 53 and 54 are fixed to an inner wall at the position in the direction opposite the horizontal direction of the bending piece 44 of the section 12b on the proximal-end side. Proximal-end portions of the coil pipes 53 and 54 are fixed to a positioning portion of the control section. Thus, the coil pipes 53 and 54 accurately applies, at the distal-end portions thereof, the amount of pulling operation applied at the proximal-end portions thereof to the bending operation wires 51 and 52.

At the bending section 12, the selective operation of the bending operation wires 51 and 52 bends only the section 12a on the distal-end side of the bending section 12 in the horizontal direction or in the left or right direction.

Similarly, a pair of bending operation wires 55 and 56 pass through non-compressing coil pipes 57 and 58, and are pierced through the bending section 12 and the flexible tube section 13 along a portion near the pivot thereof in the up and down directions.

The distal-end portions of the bending operation wires 55 and 56 are fixed at two points 55a and 56a which are apart from each other in the vertical direction of the frame member 11a on the proximal-end side of the distal-end portion 11. Proximal-end portions of the bending operation wires 55 and 56 are connected to another bending operation mechanism (manually control mechanism) (not shown) arranged in the control section, and thus the bending operation wires 55 and 56 are alternately pulled or loosened. The other bending operation mechanism (manually control mechanism) is connected to a bending operation member 23A, which will be described later.

The coil pipes 57 and 58 are fit into the bending operation wires 55 and 56, respectively. Distal-end portions of the coil pipes 57 and 58 are fixed to an inner wall at the position in the direction opposite the up and down directions of the flexible tube section 13. Proximal-end portions of the coil pipes 57 and 58 are fixed to the positioning portion of the control section.

At the bending section 12, the selective operation of the bending operation wires 55 and 56 applies the tension force throughout both the section 12a on the distal-end side and the section 12b on the proximal-end side as shown in Fig. 5, and can bend the section 12a on the distal-end side and the section 12b on the proximal-end side in the up and down directions. Referring to Figs. 4 and 5, the curvature radius of the bending section 12, which is bent in accordance with the operation of the bending operation member 23A is larger than the curvature radius of the bending section 12, which is bent in accordance with the operation of the bending operation member 23B.

As mentioned above, the bending operation wires 51 and 52 are selectively pulled, thereby adjustably bending only the section 12a on the distal-end side in the horizontal direction. Further, the bending operation wires 55 and 56 are selectively pulled, thereby adjustably bending the entire section of the bending section 12 including the section 12a on the distal-end side and the section 12b on the proximal-end side in the up and down directions.

Referring to Figs. 4 and 5, in the axial direction of the inserting portion 2, the position of the bending section 12 on the distal-end side, which is bent by the bending operation member 23A, is the same as the position of the bending section 12 on the distal-end side, which is bent by the bending operation member 23B. Further, in the axial direction of the inserting portion 2, the position of the bending section 12 on the back-end side, which is bent in accordance with the operation of the bending operation member 23A, is near the proximal-end side of the inserting portion 2, rather than the position of the bending section 12 on the back-end side, which is bent in accordance with the operation of the bending operation member 23B.

Both the bending operation wire 51 or 52 and the bending operation wire 55 or 56 are pulled, thereby entirely bending the section 12a on the distal-end side and the section 12b on the proximal-end side in the up and down directions. Further, only the section 12a on the distal-end side is bent in the horizontal direction, thereby obtaining the desired bending operation of the bending section 12. It is excessively preferable in the operation of the endoscope 1.

According to the embodiment, the length of a bent part in at least one bending direction of the bending section 12 operated by one bending operation member near the grip section 3a of the control section 3 is longer than that of a bent part in another direction of the bending section 12 operated by another bending operation member. That is, the bending range of the bending section 12, which is bent in accordance with the operation of the bending operation member 23A, is wider than the bending range of the bending section 12, which is bent in accordance with the operation of the bending operation member 23B. In other words, the length of the bend portion of the bending section 2, which is bent in accordance with the operation of the bending operation member 23A, is longer than the bending range of the bending section 12, which is bent in accordance with the operation of the bending operation member 23B. On the contrary, the bending range of the bending section 12, which is bent in accordance with the operation of the bending operation member 23B, is narrower than the bending range of the bending section 12, which is bent in accordance with the operation of the bending operation member 23A.

When the target part such as the tumor exists on the wall on the back of the plica in the lumen, which will be described later, the bending operation of the bending section 12 with the small bending radius needs to be performed so as to catch up the target part within the observing range. In this case, the bending operation is not so often used and the frequency of the bending operation is low.

According to the embodiment, in the endoscope 1 shown in Fig. 6, a bending operation member is set to the bending operation member 23B far from the grip section 3a of the control section 3. The bending operation member pulls and loosens the bending operation wire 51 or 52 which applies the tension force only to the section 12a on the distal-end side of the bending section 12. The bending operation member 23B can be used by the right hand.

On the contrary, in the endoscope 1, a bending operation member is set to the bending operation member 23A near the grip section 3a of the control section 3. The bending operation member pulls and loosens the bending operation wire 55 or 56. The thumb of the left hand which grips the grip section 3a easily reaches the bending operation member 23A.

Referring to Fig. 7, the bending directions of the section 12a on the distal-end side and section 12b on the proximal-end side of the bending section 12 operated by the bending operation member 23A are the direction for moving the observed image 61 displayed on the monitor 60 as display means in the gravity direction (up) or non-gravity direction (down) of the monitor 60.

Further, referring to Fig. 7, the bending direction of the section 12a on the distal-end side of the bending section 12 operated by the bending operation member 23B is the direction for moving the observed image 61 displayed on the monitor 60 as the display means in the horizontal direction of the monitor 60, namely, left direction or right direction.

Referring to Fig. 8, in the lumen such as the large intestine, when the target part such as the tumor exists on the back wall of the plica of the lumen, the bending section 12 of the endoscope 1 is bent at an angle of 180° or more and the treatment is performed with the reverse video image. Then, the left and right directions are distinguished so as to catch the target part within the observing range and then, selectively, the bending section 12 is small bent.

In this case, at the distal-end portion 11, referring to Fig. 9, the treatment instrument inserting channel 29 is arranged in the outer peripheral direction of the objective optical system 26. Specifically, the treatment instrument inserting channel 29 is inserted in the inserting portion 2. The bending section 12 is bent in a bending direction BO as shown in Fig. 8. The instrument channel outlet 29A is arranged in a direction NBO opposite the bending direction, with respect to the arrangement position of the objective optical system 26 as the observing optical system.

Referring to Fig. 8, the bending section 12 is bent in the bending direction BO as shown in Fig. 8. However, in the inserting portion 2, the treatment instrument inserting channel 29 runs in the direction NBO opposite the bending direction BO, with respect to the central axis of the inserting portion 2.

When the bending section 12 is viewed with the reverse video image thereof and the treatment is performed in the lumen such as the large intestine, in the endoscope 1, the bending section 12 is bendable so that the treatment instrument inserting channel 29 is close to the target part such as the tumor and the objective optical system 26 is far from the luminal wall.

When the objective optical system 26 is close to the luminal wall, the field of view is not sufficiently ensured because the objective optical system 26 is shielded by a part of the uneven luminal wall. The field of view of the lumen is easily ensured when the objective optical system 26 is apart from the luminal wall.

According to the embodiment, the two of the bending operation member 23A close to the grip section 3a of the control section 3 and the bending operation member 23B apart from it are arranged as the plurality of bending operation members 23 and, thereby, the bending section 12 is bendable in the up and down directions and the left and right directions. Further, the bending operation wire is attached to the bending section 12 so as to be bent in the diagonal direction or another direction, and the bending operation member may be arranged to be bent in the direction.

The endoscope 1 with the above-mentioned structure is connected to the light source device and the video processor. Further, the suction device, the frontward water-feeding device, and the liquid feed tank are connected to the endoscope 1 and then the endoscope 1 is used for the endoscope examination. An operator grips the grip section 3a of the endoscope 1 with the left hand as shown in Fig. 1, then, inserts the inserting portion 2 in the body cavity of the object, e.g., the large intestine, and observes the target part.

Referring to Fig. 10, the large intestine has many number of sharply winding portions between lumens A and B.

The operator operates the bending operation member 23A with the thumb of the left hand which grips the grip section 3a, and bends the bending section 12. Then, in the endoscope 1, the bending operation mechanism (manually control mechanism) pulls and loosens the bending operation wire 55 or 56 and thus the tension force is applied throughout both the ranges of the section 12a on the distal-end side and the section 12b on the proximal-end side. In the endoscope 1, the bending section 12 is bent throughout both the ranges. Since the bending section 12 is long, the inserting portion 2 easily turns out the winding part only by the bending operation without the tightening state of the bending section 12. The distal-end portion 11 of the inserting portion 2 reaches the next lumen B.

Referring to Fig. 8, the distal-end portion 11 in the endoscope 1 reaches the target part.

When the target part such as the tumor exists on the luminal wall, the operator distinguishes the left and right directions so as to catch the target part within the observing range, and selectively bends the bending section 12 with the small bending radius.

The operator operates the bending operation member 23B in such a way that the thumb of the left hand is stretched and then is bent to the inside from the outside, and the bending section 12 is bent. Referring to Fig. 9, in the endoscope 1, the bending operation mechanism (manually control mechanism) pulls and loosens the bending operation wire 51 or 52, then, the tension force is applied only to the section 12a on the distal-end side, and the bending section 12 is bent.

The operator views the observed image displayed on the monitor, and treats the target part such as the tumor by using the treatment instrument such as a forceps (not shown).

At the distal-end portion 11 in the endoscope 1, the treatment instrument inserting channel 29 is arranged in the outer peripheral direction of the objective optical system 26. The treatment instrument inserting channel 29 is close to the target part such as the tumor, and the objective optical system 26 is far from the luminal wall.

Therefore, the distal-end portion of the treatment instrument such as the clamp projected from the instrument channel outlet 29A of the treatment instrument inserting channel 29 easily reaches the target part such as the tumor, and the treatment is easy with the endoscope 1. Since the endoscope 1 entirely surveys and obtains the endoscope image in the periphery of the target part which is treated by the distal-end portion of the treatment instrument without the shielding operation of one part of the luminal wall, the observation is easy.

Advantageously, the endoscope 1 performs the preferable treating operation by selecting the bending radius upon executing the treatment with the reverse video image.

Normally, the treatment instrument inserting channel 29 is the largest in size among the channels built-in the endoscope 1, and therefore the treatment instrument inserting channel 29 is small bent and then might be buckled. However, when the treatment instrument inserting channel 29 is small bent, the treatment instrument inserting channel 29 runs out of the bending section 12 (in the outer direction of the central axis of the inserting portion 2) and, thereby, the buckling of the treatment instrument inserting channel 29 is prevented as much as possible.

The present invention is applied to the endoscope 1 as an electronic endoscope having therein the image pick-up device at the distal-end portion 11 of the inserting portion 2. Further, the present invention may be applied to an electric endoscope in which an image guide (not shown) is inserted in the inserting portion 2 and an object image light-guided by the image guide is picked-up by the image pick-up device built-in the control section 3, or a socalled optical endoscope, in which the object image light-guided by the image guide is observed by an eyepiece portion arranged onto the top portion of the control section 3.

As mentioned above, according to the embodiment, upon the treatment with the reverse video image, the endoscope, in which the preferable treatment operation is performed by selecting the bending radius, may be realized.

The present invention is not limited to the above-mentioned embodiments but can variously be modified and embodied without departing from the essentials of the present invention.

### Industrial Applicability

As described hereinabove, the endoscope in accordance with the present invention ensures good treatment work by selecting the bending radius upon the treatment with the reverse video image, so that it is applicable not only for medical use but also for industrial use.

## Claims

1. An endoscope (1) comprising:
an inserting portion (2) which is insertable in an object, the inserting portion (2) comprising a bending section (12) which is bendable in a first bending direction and a second bending direction having a curvature radius smaller than that of the first bending direction, in accordance with operator's bending operation, the second bending direction being a direction perpendicular to an axis of the bending section (12), and the first bending direction being a direction perpendicular to the second bending direction and to the axis of the bending section (12);
an observing optical system (26) which is arranged to a distal end of the inserting portion (2); and
a treatment instrument inserting channel (29) which is arranged in the inserting portion (2) and has an opening portion (29A) at the distal end of the inserting portion (2), the opening portion (29A) being configured to be positioned in a direction (NBO) opposite to a predetermined direction (BO) with respect to the arrangement position of the observing optical system (26) when an observing direction of the observing optical system (26) is reversed to have a reverse video image by bending the bending section (12) in the predetermined direction (BO) in the second bending direction.

2. An endoscope (1) according to Claim 1, further comprising:
a control section (3) having a first bending operation portion (23A) to bend the bending section (12) in the first bending direction and a second bending operation portion (23B) to bend the bending section (12) in the second bending direction,
wherein the bending range of the bending section (12) which is bent in accordance with the operation of the first bending operation portion (23A) is wider than the bending range of the bending section (12) which is bent in accordance with the operation of the second bending operation portion (23B).

3. An endoscope (1) according to Claim 2, wherein, in the axial direction of the inserting portion (2), the position on the distal-end side of the bending section (12) which is bent in accordance with the operation of the first bending operation portion (23A) is substantially the same as the position on the distal-end side of the bending section (12) which is bent in accordance with the operation of the second bending operation portion (23B).

4. An endoscope (1) according to Claim 2 or 3, wherein, in the axial direction of the inserting portion (2), the position on the back-end side of the bending section (12) which is bent in accordance with the operation of the first bending operation portion (23A) is located at a more proximal end of the inserting portion (2) than the position on the back-end side of the bending section (12) which is bent in accordance with the operation of the second bending operation portion (23B).

5. An endoscope (1) according to any one of Claims 1 to 4, wherein the bending section (12) comprises a plurality of bending pieces (31, 32, 34, 36, 38, 40 to 49) which are rotatably and continuously connected, the plurality of bending pieces are bent by the first bending operation portion (23A), and a part of the plurality of bending pieces on the distal-end side is bent by the second bending operation portion (23B).

## Patentansprüche

1. Endoskop (1) mit
einem Einführbereich (2), der in ein Objekt eingeführt werden kann, wobei der Einführbereich (2) einen Biegeabschnitt (12) umfasst, der in einer ersten Biegerichtung und einer zweiten Biegerichtung, die einen kleineren Krümmungsradius als der der ersten Biegerichtung aufweist, nach Maßgabe der Biegeoperation des Bedieners gebogen werden kann, die zweite Biegerichtung eine Richtung senkrecht zu einer Achse des Biegeabschnitts (12) ist und die erste Biegerichtung eine Richtung senkrecht zu der zweiten Biegerichtung und zu der Achse des Biegeabschnitts (12) ist;
einem optischen Beobachtungssystem (26), das an einem distalen Ende des Einführbereichs (2) angeordnet ist; und
einem Behandlungsinstrument-Einführkanal (29), der in dem Einführbereich (2) angeordnet ist und am distalen Ende des Einführbereichs (2) einen Öffnungsbereich (29A) aufweist, wobei der Öffnungsbereich (29A) so ausgebildet ist, dass er in einer Richtung (NBO) entgegen einer vorgegebenen Richtung (BO) im Hinblick auf eine Anordnungsposition des optischen Beobachtungssystems (26) positioniert ist, wenn eine Beobachtungsrichtung des optischen Beobachtungssystems (26) umgekehrt wird, um durch Biegen des Biegeabschnitts (12) in der vorgegebenen Richtung (BO) in die zweite Biegerichtung ein Umkehrvideobild zu erhalten.

2. Endoskop (1) nach Anspruch 1, das des Weiteren umfasst:
einen Steuerungsabschnitt (3), der einen ersten Biegeoperationsbereich (23A), um den Biegeabschnitt (12) in die erste Biegerichtung zu biegen, und einen zweiten Biegeoperationsbereich (23B) aufweist, um den Biegeabschnitt (12) in die zweite Biegerichtung zu biegen,
wobei der Biegebereich des Biegeabschnitts (12), der nach Maßgabe der Operation des ersten Biegeoperationsbereichs (23A) gebogen wird, größer ist als der Biegebereich des Biegeabschnitts (12), der nach Maßgabe der Operation des zweiten Biegeoperationsbereichs (23B) gebogen wird.

3. Endoskop (1) nach Anspruch 2, wobei in der axialen Richtung des Einführbereichs (2) die Position auf der Seite des distalen Endes des Biegeabschnitts (12), der nach Maßgabe der Operation des ersten Biegeoperationsbereichs (23A) gebogen wird, im Wesentlichen dieselbe ist wie die Position auf der Seite des distalen Endes des Biegeabschnitts (12), der nach Maßgabe der Operation des zweiten Biegeoperationsbereichs (23B) gebogen wird.

4. Endoskop (1) nach Anspruch 2 oder 3, wobei in der axialen Richtung des Einführbereichs (2) die Position auf der Seite des hinteren Endes des Biegeabschnitts (12), der nach Maßgabe der Operation des ersten Biegeoperationsbereichs (23A) gebogen wird, an einem proximaleren Ende des Einführbereichs (2) angeordnet ist als die Position auf der Seite des hinteren Endes des Biegeabschnitts (12), der nach Maßgabe der Operation des zweiten Biegeoperationsbereichs (23B) gebogen wird.

5. Endoskop (1) nach einem der Ansprüche 1 bis 4, wobei der Biegeabschnitt (12) eine Vielzahl von Biegeteilen (31, 32, 34, 36, 38, 40 bis 49) umfasst, die drehbar und durchgehend verbunden sind, die Vielzahl der Biegeteile durch den ersten Biegeoperationsbereich (23A) gebogen wird und ein Teil der Vielzahl der Biegeteile auf der Seite des distalen Endes durch den zweiten Biegeoperationsbereich (23B) gebogen wird.

## Revendications

1. Endoscope (1) comprenant :
une partie d'insertion (2) qui peut être insérée dans un objet, la partie d'insertion (2) comprenant une section de cintrage (12) qui peut être cintrée dans une première direction de cintrage et une deuxième direction de cintrage présentant un rayon de courbure inférieur à celui de la première direction de cintrage, conformément à l'opération de cintrage d'un opérateur, la deuxième direction de cintrage étant une direction perpendiculaire à un axe de la section de cintrage (12), et la première direction de cintrage étant une direction perpendiculaire à la deuxième direction de cintrage et à l'axe de la section de cintrage (12) ;
un système optique d'observation (26) qui est disposé sur une extrémité distale de la partie d'insertion (2) ; et
un canal d'insertion d'instrument de traitement (29) qui est disposé dans la partie d'insertion (2) et comporte une partie d'ouverture (29A) au niveau de l'extrémité distale de la partie d'insertion (2), la partie d'ouverture (29A) étant configurée pour être positionnée dans une direction (NBO) opposée à une direction prédéterminée (BO) par rapport à la position de disposition du système optique d'observation (26) lorsqu'une direction d'observation du système optique d'observation (26) est inversée pour présenter une image vidéo inversée en cintrant la partie de cintrage (12) dans la direction prédéterminée (BO) dans la deuxième direction de cintrage.

2. Endoscope (1) selon la revendication 1, comprenant en outre :
une section de commande (3) comportant une première partie d'opération de cintrage (23A) pour cintrer la section de cintrage (12) dans la première direction de cintrage et une deuxième partie d'opération de cintrage (23B) pour cintrer la section de cintrage (12) dans la deuxième direction de cintrage,
dans lequel la plage de cintrage de la section de cintrage (12) qui est cintrée conformément à l'opération de la première partie d'opération de cintrage (23A) est plus large que la plage de cintrage de la section de cintrage (12) qui est cintrée conformément à l'opération de la deuxième partie d'opération de cintrage (23B).

3. Endoscope (1) selon la revendication 2, dans lequel, dans la direction axiale de la partie d'insertion (2), la position sur le côté d'extrémité distale de la section de cintrage (12) qui est cintrée conformément à l'opération de la première partie d'opération de cintrage (23A) est sensiblement la même que la position sur le côté d'extrémité distale de la section de cintrage (12) qui est cintrée conformément à l'opération de la deuxième partie d'opération de cintrage (23B).

4. Endoscope (1) selon la revendication 2 ou 3, dans lequel, dans la direction axiale de la partie d'insertion (2), la position sur le côté d'extrémité arrière de la section de cintrage (12) qui est cintrée conformément à l'opération de la première partie d'opération de cintrage (23A) est placée au niveau d'une extrémité plus proximale de la partie d'insertion (2) que la position sur le côté d'extrémité arrière de la section de cintrage (12) qui est cintrée conformément à l'opération de la deuxième partie d'opération de cintrage (23B).

5. Endoscope (1) selon l'une quelconque des revendications 1 à 4, dans lequel la section de cintrage (12) comprend une pluralité de parties de cintrage (31, 32, 34, 36, 38, 40 à 49) qui sont connectées de manière rotative et continue, la pluralité de parties de cintrage étant cintrées par la première partie d'opération de cintrage (23A), et une partie de la pluralité de parties de cintrage sur le côté d'extrémité distale est cintrée par la deuxième partie d'opération de cintrage (23B).
